# EUROPEAN PATENT APPLICATION

(11) **EP 4 800 702 A1**
(43) Date of publication of application: **02.09.2026**
(21) Application number: 25161080.4
(22) Date of filing: 28.02.2025
(51) Int. Cl.: G16H 30/40, G06N 3/045, G06N 3/08, G06N 20/00, G16H 50/20, G16H 50/70

(54) **METHODS AND SYSTEMS FOR CLASSIFYING MEDICAL FINDINGS**

(71) Applicant: Siemens Healthineers AG, 91301 Forchheim (DE)
(72) Inventor: Hermosillo Valadez, Gerardo, West Chester, 19382 (US); Kollerathu, Varghese, 560066 Whitefield, Bengalurur (IN); Wolf, Matthias, Coatesville, 19320 (US)
(74) Representative: Siemens Healthineers Patent Attorneys

(57) **Abstract**

Computer-implemented methods and corresponding systems for providing medical findings depending on a classification are disclosed. The method comprises obtaining the medical image. The method further comprises identifying a user from a plurality of users. The method further comprises obtaining at least one medical finding in the medical image. The method further comprises providing a first trained machine learning model. The first trained machine learning model being configured to classify the at least one medical finding based on the prior actions of the identified user. The method further comprises applying the first trained machine learning model to the at least one medical finding to obtain a first classification of the at least one medical finding according to prior actions of the user. The method further comprises providing the at least one medical finding to the user depending on the first classification.

## Description

Embodiments of the present invention relate to systems and methods for providing medical findings, in particular, medical findings identified in medical images. In particular, embodiments of the present invention relate to the training of a machine learning model and to provide a trained machine learned model. In particular, embodiments of the present invention relate to the usage of trained machine learning models for medical data processing and diagnosis.

Machine learning methods and algorithms are applied widely to generate insights and/or (predictive) medical findings from medical image data. Typically, detection methods are used to process medical images acquired with an imaging unit (such as a magnet resonance system). In this regard, machine learning methods have proven very versatile in various fields of application. They are used, for instance, to support decision making and/or to provide medical diagnoses information.

However, those detection machine learning methods often provide a finding output that deviates significantly form human annotated finding outputs. Often, the detection methods are very efficient in identifying abnormalities in images. However, the result that is presented to a user often comprise so-called false positives. For example, even if detection method is showing a lesion, users, in particular clinicians, do not always accept those findings as "true" finding and may reject those because they perceive them as false positives. Users therefore hesitate to accept more challenging findings because of a perception that detection methods provide erroneous and inaccurate results. This situation arises in the healthcare business in many cases and can lead to complex, expensive and user-unfriendly diagnosis workflows.

One way of addressing this problem is to train the detection methods on specific training data and/or for specific sites.

One issue in this regard is that such models do not always generalize well. Additionally, the context and processes may vary from clinician to clinician and from site to site. As highly specialized models often cannot capture such variations in the context, this can complicate the evaluation process.

It is therefore an object of embodiments of the invention to provide methods and systems for an improved way of providing medical findings. In particular, it is an object of embodiments of the present invention to provide systems and methods which allow for a more efficient evaluation of medical findings and/or a more efficient diagnosis workflow. In particular, it is an object of embodiments of the present invention to decrease the number of false positive provided to a user in consideration of a user specific application context.

In particular, it is an object of the present invention to provide an improved computer-implemented method for providing medical findings and supporting a user/physician/radiologist/pathologist in deriving a medical diagnosis from a medical image.

This object is solved by a computer-implemented method for providing medical findings depending on a classification, a computer-implemented method for providing a trained machine learning model, a medical image detection system and corresponding computer-program products and computer-readable storage media according to the main claims. Alternative and/or preferred embodiments are object of the dependent claims.

In the following, the technical solution according to the present invention is described with respect to the claimed apparatuses as well as with respect to the claimed methods. Features, advantages, or alternative embodiments described herein can likewise be assigned to other claimed objects and vice versa. In other words, claims addressing the inventive method can be improved by features described or claimed with respect to the apparatuses. In this case, e.g., functional features of the method are embodied by objective units or elements of the apparatus.

The technical solution will be described both with regard to methods for providing medical findings depending on a classification and also with regard to methods for providing a trained machine learning model. Features and alternate forms of embodiments of data structures and/or functions for methods and systems for providing trained machine learning models can be transferred to analogous data structures and/or functions for methods and systems for providing training or test data. Analogous data structures can, in particular, be identified by using the prefix "training".

According to an aspect, a computer-implemented method for providing medical findings depending on a classification is provided. The method comprises obtaining the medical image. The method further comprises identifying a user from a plurality of users. The method further comprises obtaining at least one medical finding in the medical image. The method further comprises providing a first trained machine learning model. The first trained machine learning model is configured to classify the at least one medical finding based on the prior actions of the identified user. The method further comprises applying the first trained machine learning model to the at least one medical finding to obtain a first classification of the at least one medical finding according to prior actions of the user. The method further comprises providing the at least one medical finding to the user depending on the first classification.

The medical image data set may relate to two-dimensional image data providing two dimensions in space. Further, the medical image data set may relate to three-dimensional image data providing three dimensions in space. The medical image data set depicts a body part of a patient in the sense that it contains two- or three-dimensional image data of the patient's body part. The medical image data may, for example, be in the form of an array of pixels or voxels. Such arrays of pixels or voxels may be representative of intensity, absorption or other parameter as a function of three-dimensional position, and may, for example, be obtained by suitable processing of measurement signals obtained by a medical imaging modality. A medical imaging modality corresponds to a system used to generate or produce medical images. For example, a medical imaging modality may be a computed tomography system (CT system), a magnetic resonance system (MR system), an angiography (or C-arm X-ray) system, a positron-emission tomography system (PET system) or the like. The depicted body part of the patient in general will comprise a plurality of anatomical structures and/or organs. Taking a chest image as an example, the medical image data may show lung tissue, the rib cage, lymph nodes and others.

The medical image data set may in particular comprise histopathology slide images, in particular whole-slide images (WSI). Whole-slide images to may be two-dimensional digital images having a plurality of pixels. Whole slide images may have a size of at least 4.000 x 4.000 pixels, or at least 10.000 x 10.000 pixels, or at least 1E6 x 1E6 pixels. A whole-slide image may image a tissue slice or slide of a patient. The preparation of the tissue slices from the tissue samples can comprise the preparation of a section from the tissue sample (for example with a punch tool), with the section being cut into micrometer-thick slices, the tissue slices. Another word for section is block or punch biopsy. Under microscopic observation, a tissue slice can show the fine tissue structure of the tissue sample and, in particular, the cell structure or the cells contained in the tissue sample. When observed on a greater length scale, a whole-slide image can show an overview of the tissue structure and tissue density. The tissue may have been taken from at least one particular medical finding, e.g. a tumor the patient is suffering from. In particular, the tissue may show a manifestation of a cancerous disease of the patient, such cells of a tumor.

The preparation of a tissue slice further may comprise the staining of the tissue slice with a histopathological staining. The staining in this case can serve to highlight different structures in the tissue slice, such as, e.g., cell walls or cell nuclei, or to test a medical indication, such as, e.g., a cell proliferation level. Different histopathological stains are used for different purposes in such cases, e.g. as medical findings. The histopathological stain may characterize medical findings.

To create the whole-slide image, the stained tissue slices are digitized or scanned. To this end, the tissue slices are scanned with a suitable digitizing station, such as, for example, a whole-slide scanner, which preferably scans the entire tissue slice mounted on an object carrier and converts it into a pixel image. In order to preserve the color effect from the histopathological staining, the pixel images are preferably color pixel images. Since in the prediction both the overall impression of the tissue and also the finely resolved cell structure may be of significance, the whole slide images typically have a very high pixel resolution. The data size of an individual image can typically amount to several gigabytes.

The medical image may be associated to a medical report. The medical image and medical report may be comprised by a medical data set. The medical image may be obtained from the medical data set. A medical report may comprise structured or unstructured text in natural language. A medical report may summarize medical findings for a patient with one or more words. A medical report may comprise marked medical images to characterize medical findings. Each medical report may relate to a designated patient and/or medical data set.

Each medical finding may relate to corresponding data item in the medical data set and/or a medical image. The term "medical finding" may relate to a set and/ or a plurality of medical findings. A medical finding may indicate a certain condition or pathology of the patient. In addition, the medical finding may indicate certain workflow steps to be carried out in diagnosis and/or treatment of the patient. In other words, a medical finding may relate to any piece of information comprised in the medical data set and/or medical image such as type, characteristics and/or locations of the medical findings in the medical data set, in particular the medical image.

A medical finding may relate to an anatomical structure that differentiates the patient from other patients. Medical findings may be located within different organs of the patient (e.g., within the lung of a patient, or within the liver of a patient) or in between the organs of the patient. In particular, a medical finding may also relate to a foreign body. In particular, a medical finding may relate to a neoplasm (also denoted as "tumor"), in particular, a benign neoplasm, an in-situ neoplasm, a malignant neoplasm and/or a neoplasm of uncertain/unknown behavior. In particular, a medical finding may relate to a nodule, in particular, a lung nodule. In particular, a medical finding may relate to a lesion, in particular, a lung lesion.

A medical finding may comprise candidate medical findings. A candidate medical finding in general may indicate a potential finding for further review either by a user or a subsequent computer-implemented processes such as a classification process for verifying candidate medical findings. As such, the candidate medical findings may also comprise "false positives" that do not turn out to relate to real medical findings.

Obtaining the medical image in the framework of the application may mean that the medical image or medical data set is acquired from a healthcare information system. Thereby, the resources of the healthcare information system may be queried separately, e.g., on the basis of the target patient's ID or other suitable identifier.

According to some examples, "configured to classify, based on the prior actions of the identified user" may mean that the first trained machine learning model is configured to classify the at least one medical finding according to prior actions of the identified user. According to some examples the first trained machine learning model has (exclusively) been trained based on prior actions of the identified user (and not based on prior actions of other users of the plurality of users).

The method may comprise providing a first trained machine learning model configured to classify medical findings according to prior actions of the identified user.

In general, a trained machine learning model is configured to provide a desired or predetermined kind of output by processing a certain kind of input data. Thereby, a trained machine learning model mimics cognitive functions that humans associate with other human minds. In particular, by training based on training data, the trained machine learning model is able to adapt to new circumstances and to detect and extrapolate patterns. Other expressions for trained machine learning model may be machine-learned model, trained function, trained mapping specification, mapping specification with trained parameters, function with trained parameters, algorithm based on artificial intelligence, or machine learned algorithm.

In general, parameters of a trained machine learning model can be adapted by training so as to obtain a model update (e.g., in the form of a local update or a central update of the machine learned model at the model aggregator device). In particular, supervised training, semi-supervised training, unsupervised training, reinforcement learning and/or active learning can be used. Furthermore, representation learning can be used. In particular, the parameters of the trained machine learning model can be adapted iteratively by several steps of training.

In particular, a trained machine learning model can comprise a neural network, a support vector machine, a decision tree and/or a Bayesian network, and/or the trained function can be based on k-means clustering, Q-learning, genetic algorithms, a transformer network and/or association rules. In particular, a neural network can be a deep neural network, a convolutional neural network or a convolutional deep neural network. Furthermore, a neural network can be an adversarial network, a deep adversarial network and/or a generative adversarial network. Moreover, a neural network can comprise a transformer network.

According to an example, the first trained machine learning model comprises a machine learned and/or learnable (artificial) neural network. A neural network may comprise an input layer and an output layer. It may further comprise a plurality of layers between input and output layer. Each layer comprises at least one, preferably a plurality of nodes. Each node may be understood as a biological processing unit, e.g., a neuron. In other words, each neuron corresponds to an operation applied to input data. Nodes of one layer may be interconnected by edges or connections to nodes of other layers, in particular, by directed edges or connections. These edges or connections define the data flow between the nodes of the network. In particular, the edges or connections are equipped with a parameter, wherein the parameter is often denoted as "weight". This parameter can regulate the importance of the output of a first node to the input of a second node, wherein the first node and the second node are connected by an edge. In particular, a neural network can be trained. In particular, training of a neural network is performed based on known pairs of input and output values according to a 'supervised learning' technique, wherein the known input values are used as inputs of the neural network, and wherein the corresponding output value of the neural network is compared to the corresponding known output value. The artificial neural network independently learns and adapts the weights for the individual nodes as long as the output values of the last network layer sufficiently correspond to the known output values according to the training data. For convolutional neural networks, this technique is also called "deep learning'. The terms 'neural network' and "artificial neural network' can be used as synonyms.

Generally, the user refers to medical personnel, in particular diagnosing clinicians or radiologists. Usually, multiple users practice together and/or parallel at one site, in particular a medical institution such as a hospital. Multiple users can constitute a user group. Each user from a plurality of users may prefer different diagnosis workflows. A user characterizing user information, e.g. a user identifier, can be selected out of a list of user information, in particular by the user.

According to some examples, the step of identifying the user may comprise identifying the user based on the medical image. According to some examples, this may comprise identifying a user ID assigned to the medical image data and identifying the user based on the user ID. The user ID may comprise a username, biometric data (fingerprints, facial recognition), a code (QR-code) or multi-factor authentication.

According to one example, the method may comprise employing unique identifiers like user IDs, session tokens, or digital certificates to maintain the user's identity across different interactions and sessions. These identifiers are stored securely and are used to fetch user-specific data, preferences, and permissions, in particular medical findings and/or classifications. Additionally, algorithms and machine learning models can analyze user behavior patterns, further enhancing the accuracy of user identification and minimizing the risk of identification errors.

According to some examples, the first machine learning model has been specifically trained to classify prior medical findings according to prior actions of the user. According to some examples, the step of providing the first trained machine learning model comprises, selecting the first trained machine learning model, based on the identified user, from a plurality of trained machine learning models which respectively have been specifically trained to classify medical findings according to prior actions of an individual user (of the plurality of users).

According to some examples the plurality of users may be the group of users as herein described.

A classification generally may be conceived as a statement as to the (pathological) relevance of a medical finding, in particular in view of the respective user. The term "relevance" may be interpreted as a measure whether or not the medical finding requires further medical investigation (either by the user and/or by some other computer implemented method). Indicating the relevance may mean indicating whether the finding is pathological relevant or not. In particular, the term "relevance" may be interpreted as whether a certain user determines a finding to be (pathological) subjectively relevant or not. Further, indicating the relevance may mean indicating whether the medical finding is an actual (pathological) abnormality in the body part of the patient, in particular in the view of the user. Still further, indicating the relevance may mean indicating whether the medical finding relates to a false positive (i.e., not relating to an actual abnormality). The classification may relate to a feature comprised in the medical finding but may likewise be conceived as a classification of the medical finding as such. The classification may be binary (e.g., relevant vs. not relevant; actual abnormality vs. false positive). Further, the classification may be in the form a confidence score or likelihood for a user action with respect to the at least one medical finding.

The first classification may be conceived as a prediction if the user will report the at least one medical finding in a medical report. Further, the first classification may be conceived as a prediction that the user will accept the at least one medical finding (as true medical finding), and/or reject the at least one medical finding (as false positive) based on respective prior actions of the user for similar medical findings. In other words, the first trained machine learning model may have been trained to predict user actions with respect to (new) medical findings based on respective prior actions of the user for similar medical findings.

The term "prior actions of the user" may relate to prior classifications of medical findings by the user, in particular, manually. In particular, the term "prior actions of the user" may comprise the actions of rejecting and/or accepting (previous) medical findings, in particular, during a review of the (previous) medical findings. Additionally, the term "prior actions of the user" may include the actions of manually adding, segmenting, annotating and/or classifying (previous) medical findings by a user. In other words, by the means of the prior actions of the user, a plurality of medical findings (in particular, a set of medical findings) may be assigned to multiple subsets or classes. One subset of medical findings may comprise the medical findings considered "standard", expected and/or accepted by the user. One subset of medical findings may comprise the medical findings considered false, unexpected and/or rejected findings by the user. To put it differently, the term "prior actions of the user" may relate to a pre-labeling of medical findings of a single user. In other words, classifying the medical findings according to prior actions of the user contains an information about the subjective understanding of the user what constitutes a "real finding", e.g., a pathologically relevant abnormality.

The at least one medical finding may be provided by outputting the at least one medical finding to a user via a user interface, in particular, by displaying the at least one medical finding in a graphical user interface. Further, providing the at least one medical finding may comprise storing the at least one medical finding in an appropriate data format, e.g., together with a (first) set of medical findings. Further, providing may comprise including the at least one medical finding in an output data structure or generating an output data structure based on the at least one medical finding. According to some examples, the output data structure may be an electronical medical report.

According to some examples, the step of providing may comprise not providing (showing) the at least one medical finding to the user based on the first classification.

According to some examples, providing the at least one medical finding to the user may comprise displaying a representation to the user in a user interface. The representation may in particular comprise the at least one medical finding depending on the first classification.

By providing the at least one medical finding depending on the first classification, context-specific medical findings can be generated. One subset of medical findings can be considered as a "standard" and/or expected - corresponding to usually accepted findings - by the user. This in particular may suggest to the user that the detection and/or classification process works well. A second set of medical findings - usually rejected findings - can be optionally provided to the user, e.g., to show alternatives and/or additional information which, in some cases, may provide additional insights for a user but in other cases may comprise outliers which are not relevant for the user. To achieve this, medical findings may not be considered in an arbitrary but only in a specific manner, namely in connection with a user-specific classification and/or are only presented to the user in specific contexts (e.g. user specific complex diagnosis). This offers an individualized diagnosis workflow highlighting relevant medical findings without having to consider the rest of the detected findings on a regular basis. This enables identifying and providing medical findings in medical images in a more secure and complete manner without increasing the burden for a user to review an increased number of false positives. With that, the user is better supported to derive a medical diagnosis from a medical image.

According to some examples, based on the first classification of the medical findings a user avatar can be generated. The user avatar, representing a user's diagnostic preferences and expertise, may be generated based on the first classification, in particular medical findings that the user has previously accepted or rejected. A user avatar may comprise a machine learning model trained on training data of one specific user. A (other) user may select a user avatar. In other words, the user avatar may provide medical findings in a view of a chosen user. The provided medical findings may then be mapped to the user avatar. The user avatar may serve as a personalized representation of a medical expert.

The user avatar may be used to help clinicians to better understand and analyze medical findings. Through continuous updates and feedback from medical professionals, the avatar may evolve, ensuring it remains accurate and reflective of the latest medical information. The avatar may also depict the knowledge of an expert and/or professional no longer available and may provide a second medical opinion.

According to an aspect, obtaining at least one medical finding in the medical image further comprises providing a computer aided detection algorithm. The computer aided detection algorithm is configured to identify medical findings in medical images. Obtaining at least one medical finding in the medical image further comprises applying the computer aided detection algorithm to the medical image to generate at least one medical finding.

Computer aided detection algorithms (CAD-Algorithm) in general may refer to methods to help radiologists interpret medical images. The CAD-Algorithm may be configured to automatically identify suspicious regions, in particular medical findings, in the medical image. Here, use can be made, in particular, of the computer aided detection algorithms described in EP 4 328 855 A1.

Such suspicious regions, in particular medical findings, may contain image patterns indicative of abnormalities which may comprise cancerous growths, masses, abscesses, lacerations, calcifications, lesions and/or other irregularities within biological tissue and which can cause serious medical problems if left undetected.

In other words, the CAD-Algorithm may be a medical findings detection algorithm generally configured to detect candidate medical findings in medical images. For instance, the CAD-algorithm may have two or more stages: a detection stage for detecting potentially relevant patterns in image data and one or more classification stages for (pre)-classifying the potentially relevant patterns as medical findings (or the at least one medical finding). The potentially relevant patterns (pre-)classified as medical findings may be provided as medical findings (the at least one medical finding) to be subjected to the first trained machine learning model (which has been specifically configured for the user) in the step of applying the first trained machine learning model to the at least one medical finding.

According to some examples, in contrast to the first trained machine learning model, the CAD-algorithm is not specially adapted for a/the user, but configured to user-independently identify medical findings in medical images. According to some examples, the CAD-algorithm is different or independent from the first trained model. This may mean that the CAD-algorithm has a different architecture than the first trained model and/or has been trained in an independent training process on different training data.

In particular, findings detection algorithms may comprise one or more machine learned functions trained to detect and classify candidate medical findings if applied to a medical image. Suitable trained functions for this task include (artificial) neural networks, such as convolutional neural networks. In other words, medical findings obtained with computer aided detection are provided as input data for applying the first trained machine learning model to the medical findings depending on the identified user. In other words, the computer aided detection algorithm may provide generally classified medical findings whereas the first machine model provides a classification based on an identified user.

Obtaining the at least one medical finding may also comprise applying more than more, in particular different, computer aided detection algorithm to the medical image. One computer aided detection algorithm may for example be configured to detect lesions in medical images of a specific body region of a specific patient group.

According to a further possible aspect of the invention, the first trained machine learning model comprises the computer aided detection algorithm and a classification unit, wherein the classification unit is configured to process the output (i.e., the at least one medical finding) of the computer aided detection algorithm and to classify the output based on / according to the prior actions of the identified user, so as to determine the first classification specifically for the identified user. In particular, the classification unit comprises or consist of additional neural network layers.

An ideal CAD algorithm should be able to securely identify all actual abnormalities as findings without generating any false positives. This may sound straightforward but is very difficult to achieve in practice as this means fulfilling two conflicting requirements. At the one hand, CAD algorithms have to be highly sensible so that no potentially relevant objects remain undetected. On the other hand, a highly sensitive detection inevitably increases the likelihood of generating false positives. Additionally, even human experts do not always agree on the interpretation of medical images. These conflicting requirements and perceptions may be mitigated advantageously with the provided first trained machine learning model to classify the medical findings after the detection of the CAD algorithm based on an identified user. This, for example, allows for the CAD algorithm to be operated at a high sensitivity, while the false positives are detected by the ensuing application of the first trained machine learning model depending on prior actions of the user.

According to an example, obtaining at least one medical finding in the medical image further comprises obtaining a medical finding information from a user different from the identified user. Obtaining at least one medical finding in the medical image further comprises extracting at least one medical finding from the medical finding information.

The medical finding information preferably is an information provided by a user different from the identified user. Preferably the medical finding information comprises medical findings manually identified, segmented and/or indicated by a user. In other word instead of for example a CAD provided medical finding a manually user-created medical finding can be obtained.

According to an aspect, the first trained machine learning model is configured to classify (and/or cluster) medical findings into a first and second class. The medical findings in the first class correspond to findings previously confirmed by the identified user. The medical findings in the second class correspond to findings previously rejected (or ignored) by the user.

In the context of the present aspect, the first class is defined as a cluster or class of medical findings that have been previously confirmed as relevant by the user (as prior actions). These findings may correspond to (in the view of the identified user) actual abnormalities or pathologically significant features identified in an examined region of a patient. The second class is defined as a cluster and/or class of medical findings that have been previously rejected (or ignored) by the user (as prior actions), indicating that they are (in the view of the identified user) either false positives and/or not pathologically relevant.

The trained machine learning model has been trained on medical finding training data annotated by the identified user preferably. To put it differently, the model may be trained using a comprehensive dataset of medical images initially and findings that may have been pre-labeled by the user alone. The dataset may include examples of both relevant (accepted) and irrelevant (rejected) findings of the user. In other words, the trained machine learning model is provided during training with an understanding of what constitutes a "real finding", e.g. a pathological abnormality, according to the specific user. The differentiation between the first and second class may comprise a plurality of algorithmic assessments and/or confidence scoring, allowing for a classification into the class that the finding has the least contextual distance to.

Once trained, the trained machine learning model may process medical findings provided by a detection algorithm, in particular a CAD algorithm. By analyzing features, such as e.g. shape, size, texture, and other relevant characteristics, the trained machine learning model may assign a score to each finding. The score may reflect the assignment to a class. As an example, findings with high scores may be classified into the first class (accepted), for example indicating that they are likely to have been classified as actual abnormalities by the user. Further, findings with low scores may be classified into the second class (rejected), for example signifying that a user would have classified them as either false positives or not pathologically relevant. According to some examples, the score may be seen as a confidence score, rating, or sorting that the finding (in the view of the user) relates to an actual finding that the user would consider and, e.g., report in a medica report.

By classifying findings that have been previously confirmed as relevant by the user into the first class, the method may ensure that findings such as crucial abnormalities are prioritized for further examination. This method may therefore reduce the risk of overlooking significant medical findings. Conversely, clustering findings that have been deemed irrelevant into the second class might help minimizing the occurrence of false positives, thus preventing unnecessary follow-ups in diagnosis and/or examination. Moreover, the classification method can streamline the diagnostic workflow for medical professionals by focusing their attention on the most pertinent findings.

According to one example, the method may further comprise receiving, based on the at least one provided medical finding, a user action directed to the at least one medical finding. The user action preferably comprises accepting and/or rejecting the at least one medical finding. The method may further comprise adapting the first trained machine learning model based on the user action (as further prior action of the identified user).

The trained machine learning model may also be continuously updated based on tracked user data. The classification process may be further refined through continuous learning, where the model is periodically updated with new data and feedback from medical professionals. The first trained machine learning model may be updated and/or adjusted permanently and/or temporally.

In other words, an iterative classification method is performed. Iterative classification may comprise progressively refining the first classification through multiple iterations and/or classifications. Initial classifications may be based on a first state of the trained machine learning model, and subsequent rounds of classification may be performed using a second (or n-th) state. Each iteration may involve using different weights and/or decision boundaries. A determined iteration parameter may define the number of iterations to perform. For example, a medical finding might be classified as relevant in a first iteration, as tumor in a second iteration, as malign in a third iteration.

Iterative classification may improve overall model performance. The final classification may therefore be more reliable and precise, especially when dealing with complex and high-dimensional medical finding datasets.

According to a further aspect, the first trained machine learning model may be configured to classify the at least one medical finding by additionally incorporating information about the patient's medical history and/or previous diagnoses. The method may comprise obtaining information about the patient's medical history and/or previous diagnoses. The information about the patient's medical history and/or previous diagnoses may be defined by the user with a text prompt.

By embedding historic patient related data into the first classification, the first trained machine learning model may improve the precision of the classification of the medical findings. In particular, a classification in regard to a specific condition, disease and/or information about the patient can be provided. In other words, the provided first trained machine learning model may be configured to classify the at least one medical finding based on prior actions of the identified user and on an identified information about the patient's medical history and/or previous diagnoses.

According to some examples, the first trained machine learning model may be configured to cluster the medical findings within a feature space into distinct classes. Applying the first machine learning model the medical findings may comprise clustering into distinct classes within the feature space. Clustering the medical findings within a feature space into distinct classes may comprise extracting features and/or attributes from the medical findings. It may further comprise, mapping the features and/or attributes into a multidimensional feature space. Further, the features and/or attributes may be extracted in connection with the medical image using image processing techniques. For clustering the medical findings algorithms may be utilized, such as k-means or hierarchical clustering, to partition the feature space into two (or more) primary classes. The clustering algorithm may be configured to assign each medical finding to a class based on the similarity of its features to a centroid of the respective class. The clustering algorithm may be comprised by the first machine learning model. The first class may encompass findings exhibiting a particular set of features and/or attributes, while the second class includes features and/or attributes with the maximum median distance to features and/or attributes of the first class.

This clustering may be achieved through iterative optimization, ensuring that the intra-class similarity is maximized, and inter-class similarity is minimized. The resultant clusters provide a structured representation of the medical findings, facilitating subsequent analysis and interpretation by leveraging the inherent patterns within the medical findings.

According to an aspect, the method further comprises identifying a user group. The user is part of a user group. The method further comprises providing a second trained machine learning model configured to classify medical findings based on prior actions of the identified user group. The method further comprises applying the second trained machine learning model to the at least one medical finding to provide a second classification of the at least one medical finding according to prior actions of the group of users. The method further comprises providing the at least one medical finding to the user further depending on the second classification.

In particular the method further comprises providing a second trained machine learning model configured to classify medical findings based on prior actions of the identified user group without prior actions of the identified user.

Generally, a user group refers to a collective of medical professionals. A user group may include diagnosing clinicians, radiologists, and/or other healthcare practitioners who work together and/or in parallel at one location, such as a hospital or medical institution. Each member of the user group may have distinct preferences for diagnostic workflows and decision-making processes, cumulating in a group median and/or preference. In other words, a user group information may characterize collective actions, knowledge, and/or preferences of the group.

The user group may be identified with a group identifier, preferably selectable from a list of group identifiers. The group authentication method may involve determining, obtaining, and/or registering a group identifier and information, such as the group's name, collective data (like group usage patterns), or codes (QR-codes).

According to one example, the method may comprise employing unique group identifiers such as group IDs, session tokens, or digital certificates to maintain the group's identity across different interactions and sessions. These identifiers are stored securely and are used to fetch group-specific data, preferences, and permissions.

According to one example, advanced algorithms and machine learning models can also analyze the behavior patterns and/or preferences of a plurality of users and create, allocate and or match users to user groups.

The second trained machine learning model may comprise the same structure and/or features as the first trained machine learning model. The second trained machine learning model may differ from the first trained machine learning model particularly in the data set used for training of the second trained machine learning model.

As a consequence, the second classification is specific for the user group and not, like the first classification, specific to the identified user, only.

Aspects relating to the first trained machine learning model may also be applied to the second trained machine learning model. Providing the second trained machine learning model may be analogously implemented as providing the first trained machine learning model. Further, applying the second trained machine learning model may be analogously implemented as applying the first trained machine learning model. Further, providing the at least one medical finding to the user further depending on the second classification may be similarly implemented as providing the at least one medical finding to the user further depending on the first classification. Once trained, the second trained machine learning model may process medical findings provided by a detection algorithm, in particular a CAD algorithm as herein described, medical findings provided by radiologists and/or the medical findings provided (or forwarded) by the first trained machine learning model.

A user group may function as a cohesive unit, sharing knowledge, resources, and methodologies to enhance the accuracy and efficiency of medical diagnoses and treatment plans. By leveraging the collective expertise and experiences of multiple medical professionals, the classification and diagnosis process is significantly enriched. This extends the data available for analysis, rendering the classification more representative of real-world scenarios where variability in medical findings is common. Furthermore, a collective intelligence of the user group makes the method more resilient to user drifts and/or (concept) drift within local sites. By incorporating diverse perspectives and/or feedback from a (broader) user group, an accurate, reliable, robust method is provided, ultimately leading to improved diagnostic precision in diagnosis workflows. Still by only invoking the user group-based classification in conjunction with the user specific classification in a two-step process a sufficient level of individualization for the identified user can be attained.

According to some examples, the second trained machine learning model comprises a convolutional neural network (CNN).

The neural network comprises a plurality of layers, in particular an input layer and an output layer and optionally multiple hidden layers between input and output layer. Each layer comprises a plurality of nodes corresponding to an operation applied to input data. Each layer usually includes convolutional layers, pooling layers, and fully connected layers. Convolutional layers apply convolution operations to the input data, extracting features such as edges, textures, and patterns. Pooling layers reduce the spatial dimensions of the data, preserving essential features while reducing computational complexity. Fully connected layers integrate the extracted features to form the final output.

Convolutional neural network preferably undergoes a training process based on known pairs of input and output values using a supervised learning technique. During training, the known input values are fed into the network, and the output generated by the network is compared to the corresponding known output value. The network independently adjusts weights of the connections, aiming to minimize the difference between the predicted and actual output values. This iterative process, known as "deep learning," enables the convolutional neural network to learn and adapt to various patterns and features in the training data.

A first group of neural network layers may be applied to extract features from medical findings, in particular provided as a data set, from respective image data, text data and/or other data. Medical findings may, for instance, be given in the form of location data (coordinates), color values and/or a slice/image information. The thus extracted features like, patterns, landmarks, masks or the like may form a finding descriptor of the respective medical finding. The finding descriptors may be fed as input values to a second group of network layers which serve to determine a degree of similarity between two findings or between a finding and a reference finding and/or reference feature based on the extracted features.

The second trained machine learning may comprise a medical findings detection algorithm generally configured to detect medical findings in medical images. For instance, the second trained machine learning may have two stages: the detection stage for detecting potentially relevant findings with the findings detection algorithms. The findings detection algorithms may detect finding via patterns in image data and the classification stage for classifying the potentially relevant findings either as medical findings or as false positives to be discarded.

According to an aspect, the second trained machine learning model is configured to classify (and/or cluster) medical findings into a third and fourth class. The medical findings in the third class correspond with previous confirmed findings of the user group. The medical findings in the fourth class correspond with previous rejected findings of the user group.

In the context of the present aspect, the third class is defined as a cluster of medical findings that have been previously confirmed as relevant by the user group. These findings may align with established pathologically significant features identified across multiple users, for example indicating a consensus on their importance. The fourth class characterizes medical findings previously rejected by the user group, suggesting that these findings are either false positives or not pathologically relevant according to user group.

The second trained machine learning model may process these medical findings similar to the first trained machine learning model. The second trained machine learning model may in particular be trained on a user group training data set. The second trained machine learning model may be configured to process the medical findings by analyzing their features, such as shape, size, texture, and other relevant characteristics. The second trained machine learning model may assign a confidence score to each finding. A high confidence score for example may result in classification into the third class (accepted). A low confidence scores lead to classification into the fourth class (rejected). The confidence score of the second classification may be dependent or independent from a confidence score from the first classification.

The second trained machine learning model has been trained on medical findings annotated by the identified user group, preferably with the medical findings annotated by the identified user. To put it differently, the model may be trained using a cumulated dataset of medical images and findings that may have been pre-labeled by different users of one group. The dataset may include examples of both relevant (accepted) and irrelevant (rejected) findings of a variety of users of the user group. In other words, the second trained machine learning model is provided during training with an understanding of what constitutes a "real finding", e.g. a pathological abnormality, according to the specific user group. The differentiation between the third and fourth class may comprise a plurality of algorithmic assessments and/or confidence scoring, allowing for a classification into the class that the finding has the least contextual distance to.

Once trained, the second trained machine learning model may process medical findings provided by a detection algorithm, in particular a CAD algorithm. By analyzing features, such as e.g. shape, size, texture, and other relevant characteristics, the second trained machine learning model may assign a score to each finding. The score may reflect the assignment to a class. As an example, findings with high scores may be classified into the third class (accepted), for example indicating that they are likely to have been classified as actual abnormalities by the users of the group. Further, findings with low scores may be classified into the fourth class (rejected), for example signifying that users of the group would have classified them as either false positives or not pathologically relevant. According to some examples, the score may be seen as a confidence score, rating, or sorting that the finding (in the (cumulated) view of users of the group) relates to an actual finding that users of the group would consider and, e.g., report in a medica report.

By classifying findings that have been previously confirmed as relevant by the user group into the third class, the method may ensure that findings such as crucial abnormalities are prioritized for further examination. In particular, this may allow a user to determine medical finding that the user would have wrongly rejected. This method may therefore reduce the risk of overlooking significant medical findings. Conversely, medical findings clustered into a fourth class may be more securely deemed irrelevant, minimizing overlooked actual findings. Moreover, the classification method can streamline the diagnostic workflow for medical professionals by focusing their attention on the most pertinent findings in the specific view of the user and her or his peer group.

According to an aspect, the first trained machine learning model and the second trained machine learning model comprise different trained machine learning models. To put it differently, the first trained machine learning model and second trained machine learning model may comprise neural networks of different types, structures and/or architecture. In other words, the first trained machine learning model and the second trained machine learning model consist of different trained machine learning models. For example, the first trained machine learning model may comprise a Support Vector Machine (SVM) whereas the second trained machine learning model comprises a convolutional neural network (CNN).

SVMs are typically highly effective for small- to medium-sized datasets and for classification tasks where the decision boundary between classes is complex and requires precise, optimized separation. In other words, a SVM as first trained machine learning model would enable a robust handling of a small to medium-sized user-specific medical findings dataset. The SVM as first trained machine learning model advantageously allows for a efficient classification in high-dimensional spaces and/or in distinguishing between classes with clear margins. This might make it particularly effective for the first classification benefiting from linear separation.

CNNs are typically adept at processing and extracting features from complex, high-dimensional data, due to its convolutional layers that capture spatial hierarchies. CNNs as second trained machine learning model enable a handling of a large-scale user-group-specific medical findings dataset. In particular, by utilizing feature extraction, pattern recognition and/or the layered structure architecture of the network the CNN may allow advantageously for an efficient second classification. In other words, a CNN as second trained machine learning model may beneficially be utilized to analyze intricate features and/or information for the second classification from multiple first classifications medical findings of users of a user group.

The combination of a Support Vector Machine (SVM) and a Convolutional Neural Network (CNN) advantageously enhances the overall classification performance of the first and second trained machine learning model. By utilizing an SVM for the first classification and a CNN for the second classification the robustness, precision, accuracy and/or reliability may be improved.

According to some examples, the method further comprises providing a quality criterion, wherein the quality criterion is determined by comparing the first classification to the second classification. Determining the quality criterion may comprise applying statistical measures, metrics and/or algorithms to evaluate the consistency and/or alignment of the classifications. In other words, an individual's diagnostic assessment is compared the one made by a collective group of experts. The quality criterion may be utilized to measure the concordance and discordance between the individual and group classifications, e.g., by applying confusion matrices. The quality criterion may, e.g., be provided to the user or the group of users, e.g., in the form of a visualization.

The quality criterion may offer a robust framework for assessing individual diagnostic performance in the context of collective expertise systematically and/or the performance of the classification models in general.

According to some examples, based on the first and/or second classification and/or the quality criterion, medical findings together with the related information, may be added to a data set for training of trained machine learning models. According to some examples, adding data to the data set comprises checking if the medical findings meet a predetermined quality criterion and adding the medical findings to the data set for training if they meet the predetermined quality criterion. Based on the quality criterion outliers and/or reliable medical findings may be identified to update the trained machine learning models. By flagging outlier-findings that significantly deviate from a baseline of findings, the flagged findings are then utilized to dynamically retrain the classification model.

This iterative process ensures the trained machine learning model evolves with the dataset, enhancing precision and reliability in medical classification tasks.

According to some examples, the method further comprises identifying a site. The site may comprise multiple user groups, in particular multi-disciplinary user groups. A second trained machine learning model for each user group may be provided. Additionally, a third trained machine learning model may be configured to classify medical findings based on prior actions of the identified site may be provided. The method further comprises applying the third trained machine learning model to the at least one medical finding to provide a third classification of the at least one medical finding according to prior actions of the site. The method further comprises providing the at least one medical finding to the user further depending on the third classification. The third trained machine learning model may be provided by comparing and/or combining multiple provided second trained machine learning models. In particular, a third trained machine learning model may advantageously allow for a weighting of a classification according to different user groups.

The third trained machine learning model may comprise a comparative algorithm, such as collaborative filtering and/or matrix factorization algorithms, configured to analyze and/or compare first and/or second classifications among multiple groups so as to provide a comparative measure to the user or groups of users. The comparative measure may identify commonalities and/or discrepancies in the diagnostic approaches of a site. Cross-validation methods may be applied to verify and/or generalize across different user groups, institutes and/or medical contexts.

This example may provide a systematic classification and/or clustering model, in particular a third machine learning model. Consequently, the method may be scalable and adaptable to differed sites and/or multi-user group environments. A site-depending classification offers additional advantages compared with a user-specific and/or group-specific classification. For example, whereas groups can be defined by users of similar profession, sites can comprise servals user groups. This may lead to an interdisciplinary classification, e.g. potentially relevant for oncology diagnosis.

According to an aspect, the second trained machine learning model is only applied to medical findings clustered into the second class during the first classification of the at least one medical finding.

Advantageously the second trained machine learning model is exclusively applied to medical findings clustered into the second class to increase precision, accuracy and/or efficiency in the medical diagnosis workflow. By focusing the advanced model, in particular the second trained machine learning model, on a subset of findings, in particular of the second class, which for the user have may exhibit a more complex or ambiguous characteristics, an automated second opinion of the user's peer group may be gathered. This targeted classification ensures that the second trained machine learning model is leveraged where most needed, reducing the risk of misclassification and/or improving the overall diagnostic process. Moreover, by securely and quickly ruling out medical findings (again) with the second trained machine learning model, medical professionals can be provided with the more challenging findings with greater confidence in the system's classifications and/or recommendations.

According to a further aspect, the second trained machine learning model is applied to provide medical findings before the first trained machine learning model. The first trained machine learning model is applied to the medical findings clustered into the fourth class, in particular, in the second classification of the at least one medical finding.

According to an aspect, providing the at least one medical finding to the user based on first and/or second classification comprises at least one of:
- calculating a ranking of the at least one medical finding among further medical findings obtained from the medical image based on the at least one classification,
- generating a representation of the at least one medical finding for displaying in a user interface, the representation being configured such that the user may perceive the ranking,
- generating a representation of the at least one medical finding for displaying in a user device, the representation being configured such that the user may perceive the at least one classification,
- generating a representation of the at least one medical finding for displaying in a user device, the representation being configured such that the user may only perceive predetermined classes of the at least one classification.

The user device may in particular be a user interface. The user device and/or user interface may comprise a graphical user interface. The graphical user interface may be configured to display a representation to a user.

A ranking of the at least one medical finding may be in particular calculated for the medical findings of the first class of first classification and/or the medical findings of the third class of the second classification. The representation may be configured such that the user may perceive the medical findings of the third class of the second classification in particular.

For example, at least one medical finding could be excluded from providing to the user based on the first and/or second classification.

The calculating of the ranking may be based on a comprehensive set of features from each medical finding. The comprehensive set of features may encompass various attributes such as shape, texture, intensity, and spatial relationships. The features may be input into a ranking module, which may assign a confidence score to each finding. The confidence scores may be utilized to rank the findings in order of importance, significance and/or severity in accordance with the prior actions of the user.

Generating a representation of at least one medical finding for displaying in a user interface may involve preprocessing the medical image. Image processing algorithms may be applied to enhance the medical image with the medical findings based on the classification. Features of the medical findings may be extracted and mapped onto the medical image. However, the representation may also exclude the medical image.

The representation is configured such that the user may perceive most pertinent findings based on the classification and/or ranking, e.g. by the means of annotations and/or interactive elements to guide the user's focus on an interface.

According to an aspect in the step of providing, the at least one medical finding is only provided to the user if it was classified in the first class and/or the third class.

The provided medical findings of the first class and third class are the ones that either user or the user's peer group usually accept as "true" medical findings and "report-worthy". In other words only the medical findings are provided to the user corresponding to previously confirmed findings of the user and to findings previously rejected by the user but confirmed by the group of users. This may lead to a better contextual understanding, leading to more precise and tailored diagnosis. As a result, users and/or medical professionals receive more relevant and accurate information, without prolonging and complicating the diagnostic decision-making process.

According to an aspect, a computer-implemented method for providing a trained machine learning model configured to classify medical findings based on prior actions of a user or group of users is provided. The method for providing the trained machine learning model comprises receiving input training data. The input training data comprising medical image training data and medical report training data associated with the medical image training data. The report training data respectively comprising (previously) reported medical findings reported for the associated medical image training data by the user or group of users. The method further comprises obtaining identified medical findings from the medical image training data. In particular, medical findings are obtained by applying a (independent) computer aided detection algorithm configured to identify medical findings in medical images to the medical image training data. The method further comprises obtaining the reported medical findings from the medical report training data. The method further comprises applying the trained machine learning model to the identified medical findings so as to respectively obtain, for each of the identified medical findings, a classification result. The method further comprises comparing the classification results with the reported medical findings. The method further comprises adapting the trained machine learning model based on the comparing. The method further comprises providing the adapted trained machine learning model.

The action of having previously reported findings may be conceived as an example of prior actions according to the invention.

The input training data may comprise corresponding (training) output data. The training output data may be data the machine-learned function is expected to produce based on the input training data. The training output data may comprise verified outputs. According to some examples, the verified outputs may be verified by a (human) expert by way of medical reports.

The reported medical findings may in particular comprise accepted (and/or rejected) medical findings. The reported medical findings may in particular comprise a classification of a user and/or user group.

The input training data may be obtained from a database configured to store medical images and corresponding medical reports and/or medical findings. The database may be part of hospital information systems (HIS), radiology information systems (RIS), clinical information systems (CIS), laboratory information systems (LIS) and/or cardiovascular information systems (CVIS), picture archiving and communicating systems (PACS) or the like.

According to some examples, adapting of the trained machine learning model may comprise updating the trained machine learning model wherein one or more parameters of the trained machine learning model are changed and/or optimized, in particular, based on the comparison of the classification results. In particular, the one or more adapted parameters, may comprise one or more adapted weights and/or adapted hyperparameters of the trained machine learning model.

For an actual training (in the sense of adapting trained machine learning model) a backpropagation scheme may be used based on an appropriate cost function and using the input training data.

The classification result may refer to the output generated by the trained machine learning model processing the identified medical findings. The classification result may categorize and/or label each identified finding into classes. The classification result may indicate if the finding is "report-worthy" for the user / the group of users. The classification result may be compared to/against the actually reported medical findings extracted from the medical report training data, serving as a ground truth or reference point. The comparison may involve computing performance metrics such as accuracy, precision, recall, and other scores to quantify how the classification results match the reported findings.

By providing trained machine learning models a user- and/or group-depended classification method can be provided, aggregating, sharing and distributing the clinical knowledge gathered by one user and/or user group.

According to an aspect, obtaining reported medical findings comprises inputting the medical report training data and the corresponding medical image training data in a localizer algorithm.

The localizer algorithm is configured to extract medical findings from medical report training data and to link the medical findings to the associated image data of the medical training image data.

The localizer algorithm may identify and/or isolate medical findings in the medical report training data and match and/or map the medical findings to the image data. The linked medical image and/or matched medical findings may be referred to as localized output. The localizer algorithm may be further configured to check the localized outputs and the medical finding, in particular generated by the CAD algorithm for overlapping and/or matching based on compare metrics such as Euclidean distancing for example. The localizer algorithm may be a multimodal transformer-based model.

By effectively correlating information, the localizer algorithm enhances the overall accuracy and reliability of the trained machine learning model. In other words, the localizer algorithm bridges the gap between textual medical reports and their corresponding medical images and allows for a determination of the reporting habit of a user and/or medical professional.

According to some examples, the first trained machine learning model and/or second trained machine learning model is provided by a computer-implemented method for providing a trained machine learning model of any one of the aforementioned aspects.

According to an aspect, a utilizing of a first trained machine learning model and/or second trained machine learning provided by a method of any one of the aforementioned aspects to provide at least one medical finding to the identified user depending on the first classification and/or second classification according to the method of any one of the aforementioned aspects is suggested.

According to an aspect, a medical image detection system is provided. The medical image detection system comprises a computing unit and a user interface unit. The computing unit is configured to obtain a first and/or second classification of the medical findings in a medical image. The user interface unit configured to provide the at least one medical finding to a user depending on the first and/or second classification.

The computing unit may be realized as a data processing system or as a part of a data processing system. Such a data processing system can, for example, comprise a cloud-computing system, a computer network, a computer, a tablet computer, a smartphone and/or the like. The computing unit can comprise hardware and/or software. The hardware can comprise, for example, one or more processors, one or more memories, and combinations thereof. The one or more memories may store instructions for carrying out the method steps according to the invention. The hardware can be configurable by the software and/or be operable by the software. Generally, all units, sub-units or modules may at least temporarily be in data exchange with each other, e.g., via a network connection or respective interfaces. Consequently, individual units may be located apart from each other. Further, the computing unit may be configured as an edge device.

The interface unit may comprise an interface for data exchange with one or more other systems, e.g., via a network. The interface unit may be further adapted to interface with one or more users of the system, e.g., by displaying the result of the processing to the user (e.g., in a graphical user interface).

In particular, the medical image detection system can be configured to execute the computer-implemented method for providing medical findings depending on a classification according to the invention and its aspects. The medical image detection system are configured to execute the method and its aspects by its means, interface and/or its processor being configured to execute the respective method steps.

The advantages described in connection with the method aspects and examples may also be realized by the correspondingly configured systems' components.

According to an aspect, the present invention is directed to a computer program product comprising program elements which induce a computing unit (of a medical image detection system) to perform the steps according to one or more of the above mentioned method aspects and examples, when the program elements are loaded into a memory of the computing unit.

According to an aspect, the present invention is directed to a computer-readable medium on which program elements are stored that are readable and executable (by a medical image detection system) to perform the steps according to one or more aforementioned method aspects and examples, when the program elements are executed by the computing unit.

The realization of the invention by a computer program product and/or a computer-readable medium has the advantage that already existing providing systems can be easily adapted by software updates in order to work as proposed by the invention.

The computer program product can be, for example, a computer program or comprise another element next to the computer program as such. This other element can be hardware, e.g., a memory device, on which the computer program is stored, a hardware key for using the computer program and the like, and/or software, e.g., a documentation or a software key for using the computer program. The computer program product may further comprise development material, a runtime system and/or databases or libraries. The computer program product may be distributed among several computer instances.

Characteristics, features and advantages of the above-described invention, as well as the manner they are achieved, become clearer and more understandable in the light of the following description of embodiments, which will be described in detail with respect to the figures. This following description does not limit the invention on the contained embodiments. Same components, parts or steps can be labeled with the same reference signs in different figures. In general, the figures are not drawn to scale. In the following:
FIG. 1 schematically depicts a method for providing medical findings depending on a classification according to an embodiment;
FIG. 2 schematically depicts a method for providing medical findings depending on a classification according to an embodiment;
FIG. 3 schematically depicts an exemplary data flow diagram in connection with a method for providing medical findings depending on a classification according to an embodiment;
FIG. 4 schematically depicts a method for method for providing a trained machine learning model according to an embodiment;
FIG. 5 schematically depicts an exemplary data flow diagram in connection with a method for providing a trained machine learning model according to an embodiment;
FIG. 6 schematically depicts an embodiment of a medical detection system according to an embodiment;
FIG. 7 schematically depicts a user interface for providing an exemplary representation to a user in connection with a method for providing medical findings depending on a classification according to an embodiment.

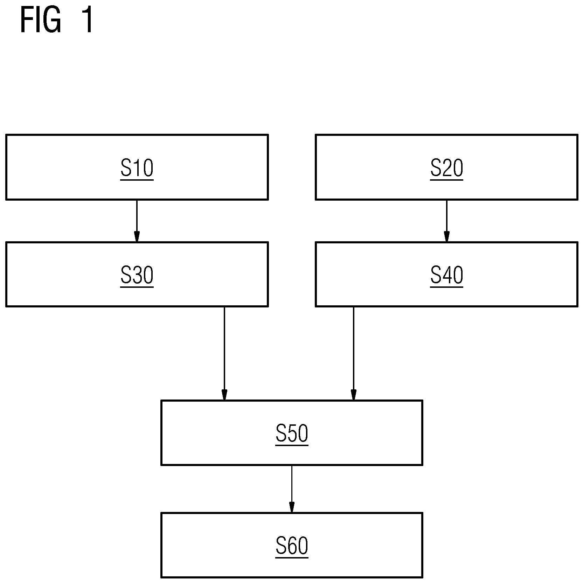

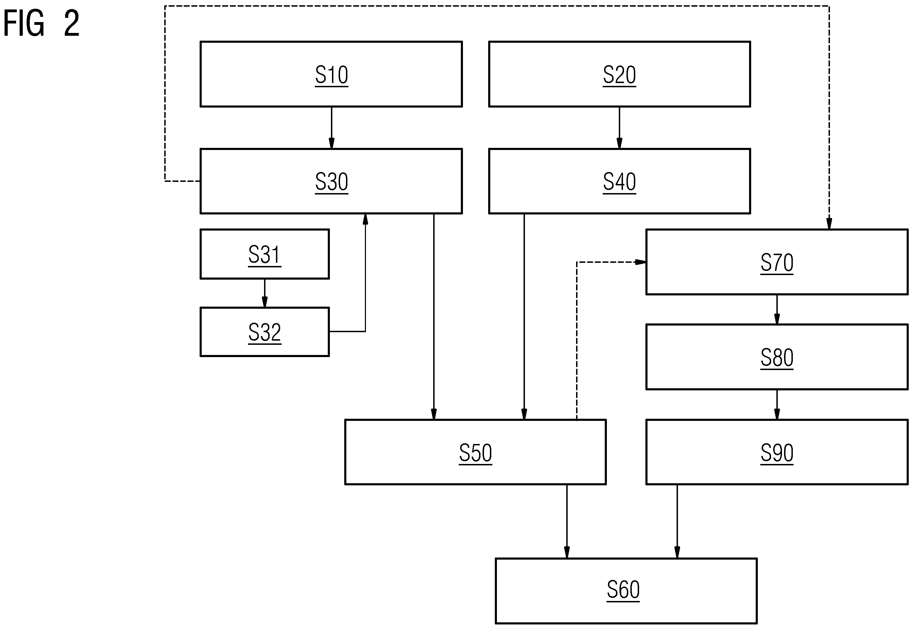

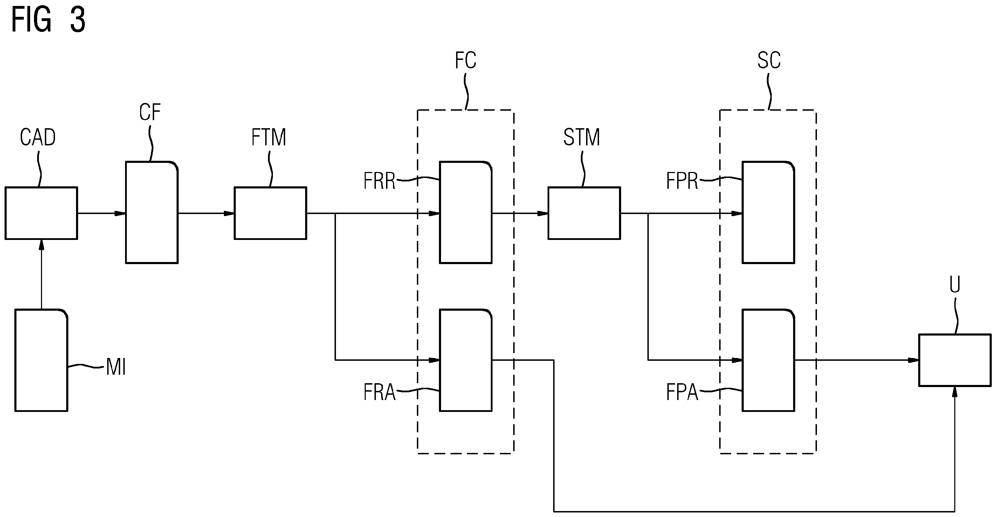

Figure 1 depicts a method for providing medical findings CF depending on a classification FC in a first embodiment. Additionally, Figure 2 shows a method for providing medical findings CF depending on a classification FC, SC in another embodiment. Corresponding data streams are illustrated in Figure 3. The expression "medical findings" in the following is to be understood to include the case of the presence of just one medical finding CF in a medical image. The method comprises several steps. The order of the steps does not necessarily correspond to the numbering of the steps but may also vary between different embodiments of the present invention. Further, individual steps or a sequence of steps may be repeated.

At S10, the medical image MI is obtained. This may involve selecting the medical image MI from a plurality of cases, e.g., stored in a database DB. The selection may be performed manually by a user U, e.g., by selecting appropriate image data in a graphical user interface GUI running in a user interface UI. Alternatively, the medical image MI may be provided to the computing unit CU by the user U by way of uploading the medical image MI to the computing unit CU. According to an example, the medical image MI has been acquired using a magnetic resonance imaging modality using a particular image weighting and magnetic resonance sequence. According to an example, the medical image MI is pulled from a database DB automatically.

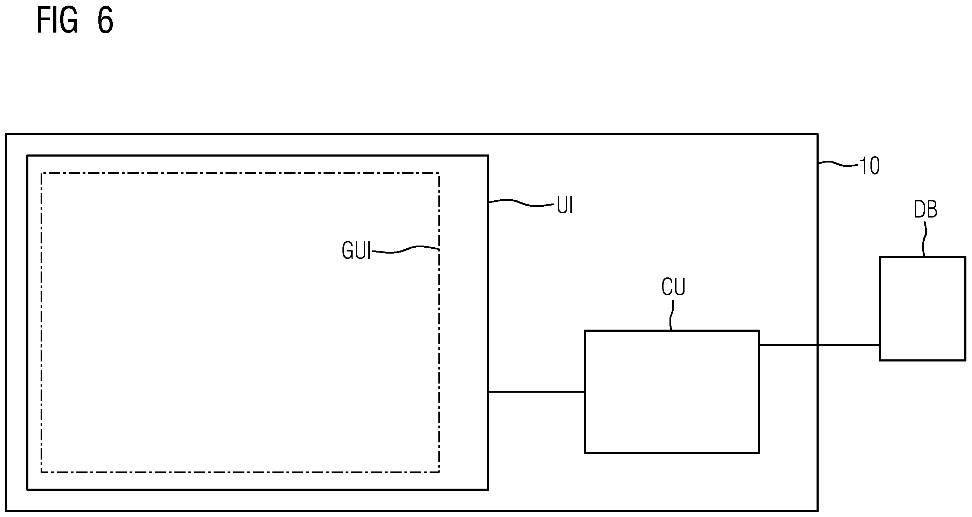

The method may be run on a computing unit CU, in particular comprised by a system 10 (for example, Figure 6) and/or a machine. The medical image MI typically contains image data and non-image data. The medical image MI is typically saved/stored in a standardized data format, DICOM for example. The medical image MI may be received at the computing unit CU. The medical image MI may be stored temporarily at the computing unit CU for processing. The obtaining of the medical image MI may comprise an automated processing of medical image data for deriving information about the patient, the imaging modality and/or other information, in particular contained in the header and/or non-image data of the medical image. Specifically, a machine learned model may be configured to process medical image data of a patient in order to obtain patient data and/or imaging modality information. S10 may also comprise obtaining information from the patient's medical history.

At S20, a user from a plurality of users is identified. In particular, the user of a system implementing the method for providing medical findings CF depending on a classification FC is identified. According to one example, a user information can be selected out of a plurality of user information by the user. The user information in other words characterizes which user from a plurality and/or group of users is to be selected. The user information can for example be determined with a user authentication method. The user authentication method can involve determining, obtaining and/or registering a user identifier, such as usernames and passwords, biometric data (fingerprints, facial recognition), or multi-factor authentication combining multiple elements for enhanced security. Once authenticated, the system employs unique identifiers like user IDs, session tokens, or digital certificates to maintain the user's identity across different interactions, user-specific data and sessions. These identifiers are stored securely and are used to fetch user-specific data, preferences, and permissions. Especially user-specific data training data may be identified and provided depending on the classifier.

At S30, at least one medical finding CF in the medical image MI is obtained. The medical finding CF in the medical image MI may be obtained automatically with a machine learned model, in particular a computer aided detection algorithm (CAD). The medical finding CF in the medical image MI may also be obtained by manual annotation of a user. The user may review the processing results of the machine learned model. For instance, a radiologist may decline or accept lesions found by the machine learned model. Further, the radiologist may add lesions not found by the machine learned model. According to other examples, a pathologist may modify a segmentation as provided by the machine learned model. The modified segmentation and/or lesions may be transferred into a medical finding CF.

Specifically, a machine learned model may be configured to process medical image data of a patient in order to detect and/or classify medical findings CF. According to some examples, the medical image data may show parts of the patient's torso, and the findings comprise lesions in the lung or liver of the patient. According to other examples, the medical image data may comprise digital pathology images of the patient and the findings relate to a segmentation of the digital pathology image according to one or more tissue types.

Optionally, at S31, the medical finding CF in the medical image MI may, in particular, be obtained with a computer aided detection algorithm (CAD). S31 may comprise providing the CAD. The CAD may be trained based on extensive datasets which include diverse examples of medical images, annotated with known findings. The CAD may be further trained based on the local data at a local site of a plurality of local sites. In other words, the CAD may be trained based on a public and/or a local training data set. In particular, the CAD may be trained independently from the first and/or second trained machine learning model FTM; STM.

Optionally, the computer aided detection may function on the basis of a prompt. The prompt may be seen as an instruction or control command for the computer aided detection algorithm. Such prompt may be generated in optional sub-step S31 on the basis of the identified user, a user group, a medical condition and/or other information. In a way, S31 may be seen as an instruction step of translating the information about the patient case / the medical image MI into a set of instructions on the basis of which the computer aided detection may provide the medical findings CF.

Optionally at S32, the CAD is applied to the medical image MI to obtain the medical findings CF. This may involve processing the medical image data through the trained CAD, which analyzes the image for medical findings CF. The CAD identifies potential medical findings CF, such as lesions or abnormal tissue, and annotates them based on the learned patterns from training data. Medical professionals may validate, modify, or add to the medical findings CF provided by the CAD. At S32 optionally, the prompt of S31 may be input into the CAD so as to trigger the generation of a data set of medical findings CF.

At S40, a first trained machine learning model FTM is provided. The first trained machine learning model FTM is configured to classify the obtained medical findings CF. The classification FC is based on the prior actions of the identified user. In particular, a first trained machine learning model FTM is selected out of a plurality of first trained machine learning models (FTM1, FTM2, ...) based on the identified user.

The first trained machine learning model FTM may be a machine learning model, such as a neural network, a language model, vision transformer, or any other suitable algorithm, that has been trained on a set of image data containing medical findings CF and corresponding user data pertaining the medical findings CF. The first trained machine learning model FTM may be hosted at a computing unit CU or at a remote server that is accessible via a network connection. The first trained machine learning model FTM may receive a data set of medical findings CF as an input and output a classification result for each medical finding CF in the data set as an output. The input may comprise image data (e.g., in the form of a cutout around the medical finding) and/or the detection result of the CAD. According to some examples, the detection result may be provided in the form of the final output of the CAD. This may comprise human readable text and image coordinates. According to some alternatives, the detection result may be provided in more abstract form, e.g., in the form of activation values of network nodes in the output layers of the CAD. Of note, according to some examples, the first (and second) machine learning model FTM is not configured to detect medical findings CF in medical image but to independent classify already detected findings - not according to a finding type but according to reporting habits of the user (or the group of users).

At S50, the first trained machine learning model FTM is applied to the medical findings CF. Hereby, a first classification FC of the medical findings CF according to prior actions of the user is obtained.

Obtaining the first classification FC may comprise providing a first trained machine learning FTM model. The plurality of medical findings CF is inputted into the first trained machine learning FTM either sequentially or at once. The first trained machine learning FTM then is configured to generate an output in the form of a classification FC for each of the medical findings CF. Specifically, the first trained machine learning FTM may be configured to take in the medical image MI (either original or processed) and an indication of a medical finding CF and output a classification FC of the medical findings CF comprised/associated within the medical image MI (e.g., in the form of an image pattern). The first classification FC represents a classification result of applying the first trained machine learning model FTM to the medical findings CF.

The first trained machine learning model FTM may extract features from the medical image MI and/or the medical findings CF that are indicative of the relevance of the medical finding CF for the user(s), such as keywords, phrases, numerical values, symbols, or any other data elements. The features may be extracted using natural language processing techniques, such as tokenization, stemming, lemmatization, part-of-speech tagging, named entity recognition, or any other suitable methods. The relevant features may be encoded in a vector or a matrix format that can be input to the first trained function FTF. The first trained function FTF may generate and/or output a probability and/or relevance score based on a set of relevant features of a medical finding CF. The probability and/or relevance score may be compared to a threshold value and/or a range to classify the medical findings CF. The classification FC into a first class and second class may represent the probability and/or relevance a user would have assigned to the medical finding CF when manually annotation the medical finding CF. The first classification FC may be temporarily stored in a digital format at the computing unit.

Optionally at S70, a user group is identified from among a plurality of user groups. The identification of the user group can be based on various attributes, including their specialization, organizational relations, past interactions with the classification model and/or system, and the types of medical findings they most frequently encounter. The user group may be identified via a user group information. Each user may be assigned to one or more user groups. The user group may be identified based on an identified user. A user may for example always be assigned to only one user group. However, a user may also be temporally assigned to a user group based on the medical diagnosis workflow at hand. According to some examples, the user group may be the user's peer group.

Optionally at S80, a second trained machine learning model STM is provided. The second trained machine learning model STM is configured to classify the obtained medical findings CF. The second classification SC is based on the prior actions of the identified user group.

The second trained machine learning model STM may generally be configured in the same way as the first trained machine learning model FTM - with the difference that it has been trained to classify medical findings CF according to prior actions of a plurality of users and not just one individual user. The second trained machine learning model STM may be a machine learning model, such as a neural network, or any other suitable algorithm, similar and/or equal to the first trained machine learning model FTM. The second trained machine learning model STM that has been trained on a set of image data containing medical findings CF and corresponding group user data. The second trained machine learning model STM may be hosted at a computing unit CU or at a remote server that is accessible via a network connection. The second trained machine learning model STM may receive classified medical findings CF as an input and output a second classification SC as an output. The second trained machine learning model STM may also receive a data set of medical findings CF as an input and output a data set containing a second classification SC for each medical finding CF as an output.

The second trained machine learning model STM may in particular only be applied to the classified output medical findings CF of the first machine trained model FTM. The second trained machine learning model STM may in particular only be apply to the second class of medical findings CF classified with the first machine trained model FTM.

Optionally, at S90, the second trained machine learning model STM is applied to the medical findings. Hereby a second classification SC of the medical findings CF according to prior actions of a user group is obtained.

The second trained machine learning model STM may extract features from the medical image MI and/or the medical findings CF that are indicative of the relevance of the medical finding CF to the user group. The second trained function STM may generate and/or output a probability and/or relevance score based on a set of relevant features of a medical finding CF. The probability and/or relevance score may be compared to a threshold value and/or a range to classify the medical findings CF. The classification SC into a third class and forth class may represent the probability and/or relevance of the medical finding CF to a user group. According to some examples, the third class may correspond to medical findings CF which a group of users would have further considered when manually annotating the medical findings CF. The fourth class may correspond to medical findings CF which a group of users would have not further considered and/or rejected when manually annotating the medical findings CF.

The second classification SC may be temporarily stored in a digital format at the computing unit CU.

The second trained machine learning model STM may in particular only be apply to the classified output medical findings FRA, FRR of the first machine trained model FTM. The second trained machine learning model STM may in particular only be apply to the second class of medical findings FRR classified with the first machine trained model FTM.

At S60, the medical findings CF are provided to the user depending on the classifications FC, SC. This may involve showing the medical findings CF, in particular as a representation, depending on the first classification FC and /or second classification SC in a user interface UI, e.g., in a suitable graphical user interface GUI. An example of a representation in a user interface UI is given in Figure 6. S60, providing the medical findings CF may comprises several sub steps. For example, S60 may also comprise providing medical findings CF based on the first and/or second classification FC, SC to further processing methods and/or algorithms.

At S61, the at least one medical finding CF is provided to the user based on first and/or second classification FC, SC. This comprises calculating, based on the at least one classification FC, SC a ranking of the at least one medical finding CF among further medical findings CF obtained from the medical image MI. The ranking may be additionally based on other information, such as patient information and/or the medical image MI. The ranking may be calculated by based on the provided classification, e.g. a score derived from the first and second classification FC, SC. According to other examples, the ranking may be calculated by a, in particular additional, trained machine learning model. Further, the trained machine learning model for calculating a ranking may be configured to calculate the ranking only based on the provided classification, e.g. a score derived from the first and second classification FC, SC.

At S62, a representation RE of the at least one medical finding CF for displaying in a user interface UI is generated. The representation RE is being configured such that the user U may perceive the ranking. A representation RE of the medical finding CF based on the first and/or second classification FC, SC may be generated. For example, the medical finding CF may be sorted based on the ranking. In particular, a representation RE comprising a list and/or another suited item to represent the medical findings CF in a determined order and/or relevance.

According to the one example the representation RE comprises the medical image MI, such as a radiological or a pathological medical image for example, and at least one information about the relevance of medical findings CF, in particular a classification FC, SC. The representation RE may also be an abstract representation of the classification FC, SC of the medical finding CF. In other words, the representation RE may exclude the medical image MI.

At S63, a representation RE of the at least one medical finding CF for displaying in a user interface UI is generated. The representation RE being configured such that the user may perceive the at least one classification FC; SC.

The first and/or second classification FC, SC are used to generate one or more appropriate representations RE of the medical image MI for displaying to a user U in the user interface UI. This may involve determining the type of representations RE coming into question for the medical finding CF depending on the first and/or second classification FC, SC and selecting and processing suited image data from the medical image MI.

At S64, a representation RE of the at least one medical finding CF for displaying in a user interface UI is generated. The representation RE being configured such that the user may only perceive predetermined classes of the at least one classification FC, SC.

A data flow diagram of the method for providing medical findings CF depending on a classification FC, SC is schematically depicted in **Figure 3****.** The data flow diagram may illustrate the methods shown in Figure 1 and 2.

The medical findings CF may be obtained by inputting a medical image MI into a computer aided detection algorithm CAD. For classification, medical findings CF may be received by a first trained machine learning model FTM. The first trained machine learning model FTM classifies the medical findings CF into a first class FRR, corresponding to medical findings CF typically approved by a user U (e.g. radiologist), or a second class FRA, corresponding to findings typically rejected by the user U. The first class FRR and second class FRA form a first classification FC. The findings of the second class FRA may be received by a second trained machine learning model STM. The second machine learning model FTM classifies the medical findings FRA, FRR initially classified by the trained machine learning model FTM again. The second machine learning model STM classifies the medical findings CF into a third class FPR, corresponding to medical findings CF typically approved by a user group (e.g. radiology department), and a fourth class FPA, corresponding to findings typically rejected by the user group. The third class FPR and fourth class FPA form a second classification SC The medical findings FRA, FPA of the first and third class are provided to the user U.

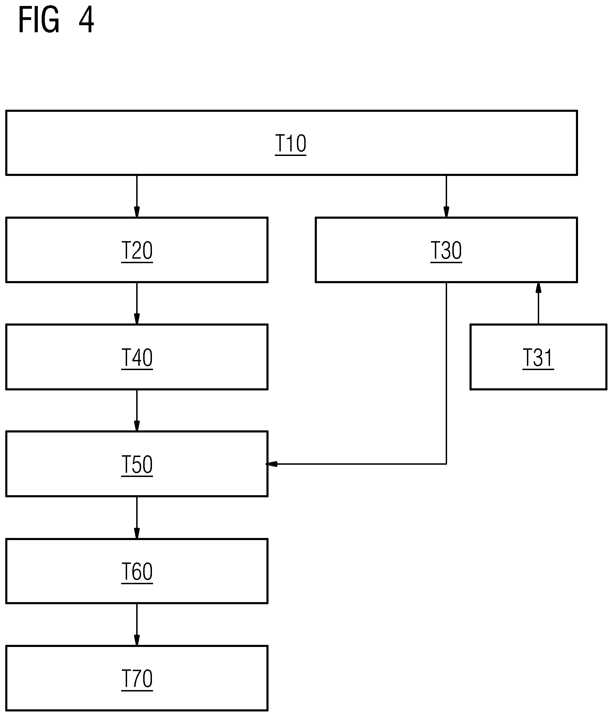

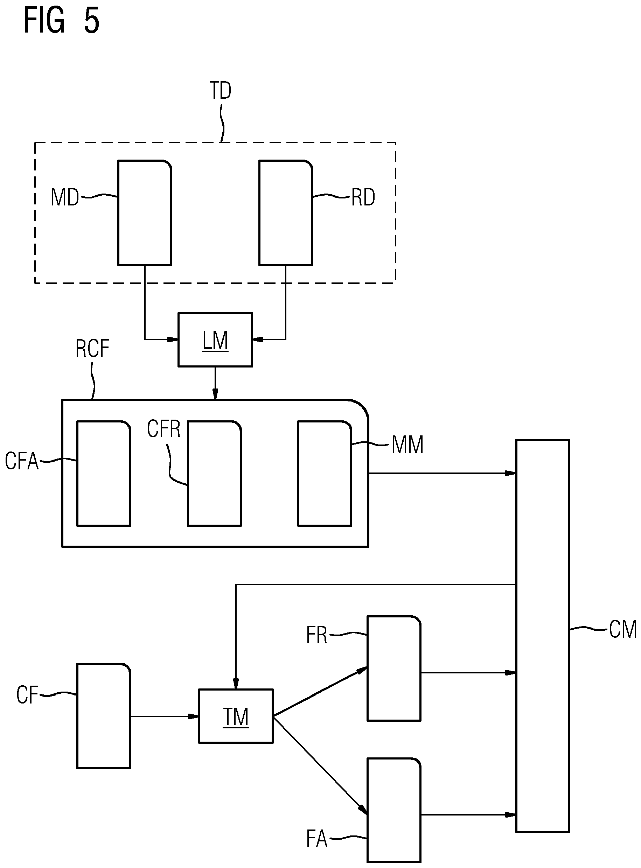

**Figure 4** depicts method for providing a trained machine learning model TM (also machine learning model TM) configured to classify medical findings CF based on prior actions of a user or group of users. Corresponding data streams are illustrated in **Figure 5****.**

The method comprises several steps. The order of the steps does not necessarily correspond to the numbering of the steps but may also vary between different embodiments of the present invention. Further, individual steps or a sequence of steps may be repeated.

At T10, the input training data TD is obtained. The input training data TD comprises medical image training data MD and related medical report training data RD. This may involve selecting the training data set form a plurality of data sets. The input training data TD may be stored in a database. Alternatively, the input training data TD may be provided to a computing unit by way of uploading a data set to the computing unit. According to an example, the input training data TD may comprise a magnetic resonance medical image annotated with classified medical findings.

The input training data TD may be stored temporarily at a computing unit for training. In particular, input training data TD is deleted from the computing unit and/or not referenced anymore after training. The obtaining of the input training data TD may comprise an automated processing of input training data TD. However, this may include for example T20 and T30.

At T20, the identified medical findings CF are obtained from the medical image training data TD. In particular, the medical findings CF are determined by applying a computer-aided detection (CAD) algorithm to medical image data of the input training data TD. The CAD algorithm may be comprised by a localizer module and/or algorithm LM. The CAD algorithm may be a trained machine learning model. The parameters and weights of the CAD algorithm may be frozen and/or defined as non-editable in the training of the trained machine learning model TM.

At T30, the reported medical findings RCF are obtained. This may comprise the application (T31) of a localizer algorithm LM. The reported medical findings RCF may be also extracted from the input training data TD, in particular the medical report training data RD. For example, the input training data TD is processed by a trained model, in particular a Large Language model (LLM) to extract clinically relevant findings from medical reports.

The input training data TD may also be obtained by applying a localizer algorithm LM to inputted medical image training data MD and related medical report data RD. The localizer algorithm LM may extract the reported medical findings RCF from the inputted data and/or may extract links, context and/or in particular a classification. In other words, by applying the localizer algorithm LM to input training data TD a user-specific data set for training may be determined. The localizer algorithm LM may determine the reported medical findings RCF based on the fact that they have been reported. Based on that, the localizer algorithm LM may sort the identified medical findings RCF into classes: identified medical findings which have been reported CFA (i.e., which can be identified in the reported medical findings) are findings the user(s) typically accept(s); identified medical findings which have not been reported CFR (i.e., which cannot be identified amongst the reported medical findings) are findings, the user(s) typically reject(s). Transferred to the desired behavior of the first machine learning model FTM, the first ones should be classified into the first class FC by the first machine learning model FTM, while the second ones should be classified into the second class SC. That way, the results of the localization algorithm LM may be used as a ground truth for the ensuing training of the first machine learning model FTM. The same scheme may be used for the second machine learning model STM with the difference that the training data comprises medical reports from users of the entire user group and not just from an individual user.

At T40, the machine learning model TM, preferably pretrained, is applied to the identified medical findings CF. The machine learning model TM provides a classification of the medical findings CF. The classification preferably comprises two classes, findings rejected FR and findings accepted FA. The classification initially may depend on the dataset used for the pretraining of the machine learning model TM.

At T50, the provided classification of the identified medical findings CF by the machine learning model TM, findings rejected FR and findings accepted FA, is compared to the reported medical findings RCF, provided in particular by the localizer algorithm. The classifications of the identified medical findings CF and the reported medical findings RCF may preferably be compared with a comparator module CM. The comparator module CM may be configured to determine a discrepancy between the classifications (in particular between the first/third class and the reported medical findings) and generate a feedback information for the machine learning model TM. The comparator module CM may be configured to analyze the classification of the machine learning model TM on the basis of the reported medical findings RCF as ground truth.

At T60, the machine learning model TM may be adapted based on a feedback information of the compare module CM. In particular the parameters and/or weights of machine learning model TM may be adapted based on the feedback information of the compare module CM. The adaptation of the machine learning model TM may be implemented iteratively. The machine learning model TM may be adapted in the training process until a determined quality criterion (e.g., a sufficient percentage of correct classifications in view of the actually reported medical findings RCF) is met.

According to some examples, the training may happen on the fly, e.g., when a user reviews the processing results of a CAD algorithm. For instance, a radiologist may decline or accept lesions found by a trained machine learning model TM. Further, the radiologist may add lesions not indicated, in particular by a CAD. According to other examples, a pathologist may modify a medical finding as provided by a CAD and/or as classified by the trained machine learning model TM. The user inputs may be used as ground truth for further optimizing, that is, training the trained machine learning model TM.

At T70, the trained machine learning model TM is provided. The machine learning model TM for example may be stored in a database and/or computing unit. Providing the trained machine learning model TM may comprise outputting a parameter and/or weights information to configure a machine learning model TM accordingly.

Figure 6 depicts an embodiment of a medical image detection system 10. The medical image detection system 10 may be capable of providing medical findings CF depending on a classification. The medical image detection system 10 may provide the means, in particular a computing unit CU, to perform one or more methods according to Figures 1 to 5. The medical image detection system 10 is adapted to perform the methods according to one or more embodiments, e.g., as further described with reference to Figures 2 to 6.

The medical image detection system 10 comprises a computing unit CU and a user interface unit UI. The computing unit CU and a user interface unit UI may be connected via a network. The medical image detection system 10 may be interfaced with a database DB. The computing unit CU is generally configured to control, coordinate and steer the classification process in medical image detection system 10. The interface unit UI may be generally configured to provide information to a user. The interface unit UI may be connected to the computing unit CU. The trained machine learning models FTM, STM can be conceived as central models which are administrated by the computing unit CU. The computing unit CU may be configured to administrate further trained models either at the computing unit CU or at connected units via e.g. a control prompt or command.

The computing unit CU may be hosted on a server, which may be a cloud server or a local server. However, the computing unit CU also may be implemented using any other suitable computing device(s). Further, the computing unit CU have access to a database DB which is configured for centrally storing medical data.

The computing unit CU may comprise one or more processors and a working storage. The one or more processor(s) may include, for example, one or more central processing units (CPUs), graphics processing units (GPUs), and/or other processing de-vices. Computing unit CU may further comprise a micro-controller or an integrated circuit. Alternatively, computing unit CU may comprise a real or virtual group of computers like a so called 'cluster' or 'cloud'. The working storage may include one or more computer-readable media such as a RAM for temporally loading data, e.g., data from the database DB. The working storage may further store information accessible by the one or more processors, for performing method steps according to one or more embodiments herein described.

A sub-unit of the computing unit CU may be a management module or unit configured for controlling and administrating the training of the trained machine learning models TM in the medical image detection system 10. The sub-unit may trigger the update of trained machine learning models TM in the medical image detection system 10, once e.g. a new updated training data set is available.

The interface unit UI (also user interface) can include any suitable components for providing the medical findings CF to a user, in particular a graphical user interface GUI. The interface unit IU, in particular a graphical user interface GUI, may display information, in particular classified medical findings, with a defined setting. Multiple interface unit UI may be comprised and/or connected to the medical image detection system 10. For example, the interface unit UI, in particular a graphical user interface GUI, may comprise a Monitor or any suitable other display device.

The interface unit UI can include any suitable components for interfacing with one or more networks, including, for example, transmitters, receivers, ports, controllers, or other suitable components. The computing unit CU may exchange information with one or more other system via the interface unit UI. Any number of systems can be connected to the system 10 over the interface unit UI.

The database DB may be realized as a cloud storage. Alternatively, the database DB may be realized as a local or spread storage, in particular, within the premises of the computing unit CU. The database CDB is configured to store training data TD.

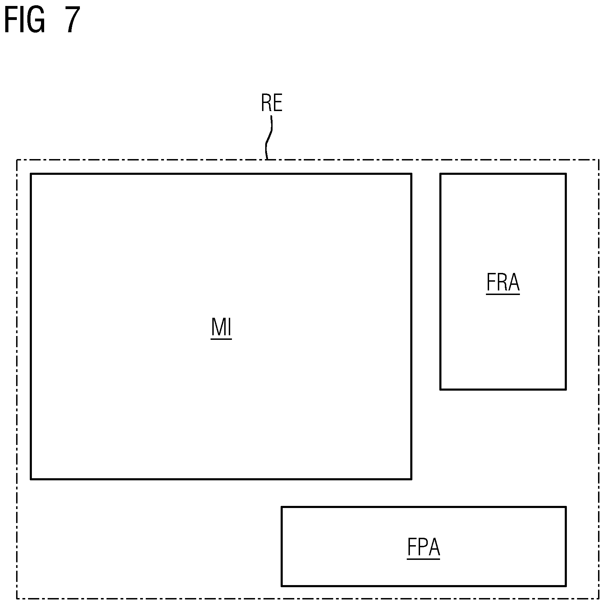

**Figure 7** schematically depicts an (graphical) user interface UI for displaying an exemplary representation to a user. The representation is generated on a user interface UI, in particular of a medical image detection system 10 of Figure 6, in connection with a method for providing medical findings CF depending on a classification FC, SC.

Figure 7 depicts an example of a representation RE. The representation RE is configured such that the user may perceive the at least one classification FC, SC and/or a ranking of the medical findings CF. The representation RE may comprise a medical image MI. The medical image MI may be the obtained medical image. However, the medical image MI may also comprise an artificially generated image and/or a reference image and/or an annotated image. The classified medical findings FRA, FPA may be display in distinct locations of the representation RE. The classified medical findings FRA, FPA may be displayed in a list, in particular sorted by classes of a first and/or second classification FC, SC and/or a ranking. The classified medical findings FRA, FPA may also be displayed together with the medical image MI. For example, the medical image MI may comprise markers at different location indicating the classified medical findings FRA, FPA. The classification FC, SC may be indicated by a text, symbol or any other suitable way.

The representation RE may comprise a generated textual summary and/or a synthetic representation based on the classifications FC, SC of the medical findings CF. For instance, the textual summary may be automatically generated by applying yet another trained function. The representation RE may optionally comprise augmentation data. For instance, the size and location of nodules may be slightly varied based on the classifications FC, SC. Further, descriptions of nodules may be added while the description of other nodules may be deleted based on the classifications FC, SC and/or ranking of the medical findings based on the classifications FC, SC.

Wherever meaningful, individual embodiments or their individual aspects and features can be combined or exchanged with one another without limiting or widening the scope of the present invention. In particular, while the systems and methods have been described in the description of embodiments with reference to medical use cases including the processing of medical data this is not to be construed as limiting the claims scope as the concepts, aspects, and examples are applicable to all kinds of data. If not indicated otherwise, brackets denote optional language, optional features, or options in general.

Advantages which are described with respect to one embodiment of the present invention are, wherever applicable, also advantageous to other embodiments of the present invention.

Independent of the grammatical term usage, individuals with male, female or other gender identities are included within the term.

## Claims

1. Computer-implemented method for providing medical findings (CF) depending on a classification, the method comprising:
obtaining (S10) a medical image (MI),
identifying (S20) a user from a plurality of users,
obtaining (S30) at least one medical finding (CF) from the medical image (MI),
providing (S40) a first trained machine learning model (FTM) configured to classify,
based on prior actions of the identified user, the at least one medical finding (CF),
applying (S50) the first trained machine learning model (FTM) to the at least one medical finding (CF) to obtain a first classification (FC) of the at least one medical finding (CF) according to prior actions of the user,
providing (S60) the at least one medical finding (CF) to the identified user depending on the first classification (FC).

2. Method according to claim 1,
wherein the obtaining (S30) at least one medical finding (CF) in the medical image (MI) comprises:
providing (S31) a computer aided detection algorithm (CAD) configured to identify medical findings (CF) in medical images (MI),
applying (S32) the computer aided detection algorithm (CAD) to the medical image (MI) to generate at least one medical finding (CF).

3. Method according to any one of the preceding claims,
wherein the first trained machine learning model (FTM) is configured to classify medical findings (CF) into a first and a second class, and
medical findings in the first class (FRA) correspond to previously confirmed findings of the identified user and medical findings in the second class (FRR) correspond to previously rejected findings of the identified user.

4. Method according to any one of the preceding claims, further comprising:
identifying (S70) a user group, wherein the user is part of the user group,
providing (S80) a second trained machine learning model (STM) configured to classify medical findings (CF) based on prior actions of the identified user group,
applying (S90) the second trained machine learning model (STM) to the at least one medical finding (CF) to provide a second classification (SC) of the at least one medical finding (CF) according to prior actions of the identified user group, and
wherein providing (S60) the at least one medical finding (CF) to the user is further depending on the second classification (SC).

5. Method according to claim 4,
wherein the second trained machine learning model (STM) is configured to classify medical findings (CF) into a third and a fourth class,
wherein medical findings in the third class (FPA) correspond to previously confirmed findings of the user group and medical findings in the fourth class (FPR) correspond to previously rejected findings of the user group.

6. Method according to claim 3 in combination with any one of claims 4 or 5,
wherein the second trained machine learning model (STM) is only applied to medical findings classified into the second class (FRR) by the first trained machine learning model (FTM).

7. Method according to any one of the preceding claims,
wherein providing (S60) the at least one medical finding (CF) to the user based on first and/or second classification (FC, SC) comprises at least one of:
calculating (S61), based on the first and/or second classification (FC, SC) a ranking of the at least one medical finding among further medical findings (CF) obtained from the medical image, and
generating (S62) a representation of the at least one medical finding for displaying in a user interface, the representation being configured such that the user may perceive the ranking;
generating (S63) a representation of the at least one medical finding for displaying in a user device, the representation being configured such that the user may perceive the first and/or second classification (FC, SC); and/or
generating (S64) a representation of the at least one medical finding for displaying in a user device, the representation being configured such that the user may only perceive predetermined classes of the first and/or second classification (FC, SC).

8. Method according to any one of claims 3 to 7,
wherein, in the step of providing (S60) the at least one medical finding (CF), the at least one medical finding is only provided to the user if it was classified in the first class (FRA) and/or the third class (FPA).

9. Method according to any one of claims 4 to 8,
wherein the first trained machine learning model (FTM) and second trained machine learning model (STM) comprise different trained machine learning models.

10. Computer-implemented method for providing a trained machine learning model (TM) configured to classify medical findings (CF) based on prior actions of a user or group of users, comprising:
receiving (T10) input training data (TD) comprising medical image training data and medical report training data associated with the medical image training data, the report training data respectively comprising reported medical findings (RCF) reported for the associated medical image training data by the user or group of users,
obtaining (T20) identified medical findings (CF) from the medical image training data, in particular by applying a computer aided detection algorithm configured to identify medical findings (CF) in medical images to the medical image training data,
obtaining (T30) the reported medical findings (RCF) from the medical report training data,
applying (T40) the trained machine learning model (TM) to the identified medical findings (CF) so as to respectively obtain, for each of the identified medical findings, a classification result,
comparing (T50) the classification results with the reported medical findings,
adapting (T60) the trained machine learning model based on the comparing (T50), and providing (T70) the adapted trained machine learning model.

11. Method according to claim 10,
wherein obtaining (T30) reported medical findings (RCF) comprises inputting (T31) the medical report training data and the corresponding medical image training data in a localizer algorithm,
wherein the localizer algorithm (LM) is configured to extract medical findings (CF) from medical report training data and to link the medical findings to the associated image data of the medical training image data.

12. Utilizing a first trained machine learning model (FTM) and/or second trained machine learning (STM) provided by a method of any one of the claims 10 to 11 to provide at least one medical finding (CF) to the identified user depending on the first classification (FC) and/or second classification (SC) according to the method of any one of claims 1 to 9.

13. A medical image detection system (10), comprising:
a computing unit (CU) configured to obtain a first and/or second classification (FC, SC) of the medical findings (CF) in a medical image (MI) according to any one of claims 1 to 9,
a user interface unit (UI) configured to provide the at least one medical finding (CF) to a user depending on the first and/or second classification (FC, SC).

14. Computer program product comprising program elements which induce a computing unit (CU) to perform steps of the method according to any of claims 1 to 11 when the program elements are loaded into a memory of the computing unit (CU).

15. Computer-readable medium on which program elements are stored that are readable and executable by a computing unit (CU) to perform steps of the method according to any of claims 1 to 11 when the program elements are executed by the computing unit (CU).
